(19) Europäisches Patentamt
European Patent Office
Office européen des brevets

(11) EP 2 325 167 A1

(12) EUROPEAN PATENT APPLICATION
published in accordance with Art. 153(4) EPC

(43) Date of publication:
**25.05.2011 Bulletin 2011/21**

(21) Application number: **09802909.3**

(22) Date of filing: **27.07.2009**

(51) Int Cl.:
***C07C 269/00*** *(2006.01)* ***C07C 271/12*** *(2006.01)*
***C07C 271/44*** *(2006.01)* ***C07B 61/00*** *(2006.01)*

(86) International application number:
**PCT/JP2009/063337**

(87) International publication number:
**WO 2010/013666 (04.02.2010 Gazette 2010/05)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **28.07.2008 JP 2008193002**
**30.10.2008 JP 2008279508**

(71) Applicant: **Ube Industries, Ltd.**
**Ube-shi**
**Yamaguchi 755-8633 (JP)**

(72) Inventors:
- **FUKUTA, Yukimasa**
**Ube-shi,**
**Yamaguchi 755-8633 (JP)**
- **YAMAMOTO, Yasushi**
**Ube-shi,**
**Yamaguchi 755-8633 (JP)**
- **MIYATA, Hiroyuki**
**Ube-shi,**
**Yamaguchi 755-8633 (JP)**

(74) Representative: **Wiedemann, Peter et al**
**Hoffmann - Eitle**
**Patent- und Rechtsanwälte**
**Arabellastrasse 4**
**81925 München (DE)**

# (54) METHOD FOR PRODUCING CARBAMATE COMPOUND

(57) A task of the present invention is to provide a process for preparation a carbamate compound using a carbonic acid ester and an amide compound in the presence of a basic compound(s), wherein the process is a novel industrial method which is advantageous in that the reaction rate is faster than conventional and the by-produced ester compound can be recovered, and the present invention is directed to a method comprising reacting an amide compound represented by the formula (1) with a carbonic acid ester represented by the formula (2) in the presence of a basic compound(s) to obtain a carbamate compound represented by the formula (3).

EP 2 325 167 A1

## Description

FIELD OF THE INVENTION

The present invention relates to a novel industrial process for preparation of carbamate compounds using a carbonic acid ester and an amide compound in the presence of a basic compound(s).

BACKGROUND ART

A carbamate compound, shortened carbamate is a useful compound as a raw material or an intermediate in the synthesis of medical and agricultural chemicals, fine chemicals and polymers. Particularly, the carbamate compound is a useful compound as an intermediate of an isocyanate compound which has widely been used as a raw material for polyurethane.

The isocyanate compound, which has widely been used as a raw material for polyurethane, has been industrially produced by using a reaction of phosgene and amine. However, phosgene is highly toxic and corrosive, and hence poses problems in that, for example, phosgene is not easy to handle, that phosgene causes corrosion of the apparatus, and that the by-produced hydrochloric acid must be treated, and therefore the development of a method for industrially producing an isocyanate compound as a substitute for the method using phosgene has been desired for a long time.

In recent years, as a safe and economical process for preparation of an isocyanate compound, a method in which an isocyanate compound is obtained by thermal decomposition of a carbamate compound is expected (see, for example, patent document 1).

Conventionally, with respect to the method for obtaining a carbamate compound, there have been well known three types of methods, i.e., methods using carbon monoxide, carbon dioxide and a carbonic acid ester, respectively, as a carbon source which can be a substitute for phosgene.

First, as examples of the methods using carbon monoxide, there are a method in which a carbamate compound is produced by oxidative carbonylation of an amine using palladium (see, for example, non-patent document 1) and a method in which a carbamate compound is produced by reductive carbonylation of a nitro compound (see, for example, patent document 2). However, in these methods, an expensive noble metal must be used as a catalyst, and the reaction must be performed under high-pressure conditions, and therefore the cost is increased and special facilities for the production are required, and thus these methods are not suitable for industrial process. Further, in the method using a nitro compound described in patent document 2, three molecules of carbon monoxide are necessary for synthesizing one molecule of the carbamate compound, and this method is not efficient.

As an example of the method using carbon dioxide as a carbon source, there is a method in which a carbamate formed from amine and carbon dioxide is reacted with an alcohol to produce a carbamate compound (see, for example, non-patent document 2). However, also in this method, high-temperature and high-pressure conditions are required and further, for obtaining a carbamate compound in high yield, an expensive dehydrating agent must be used in the stoichiometric amount or more, and thus there have been many problems about the production to be solved.

As an example of the method using a carbonic acid ester as a carbon source, there has been known a method in which a carbamate compound is produced from dimethyl carbonate and an amine (see, for example, patent documents 3 and 4).

The dimethyl carbonate used in this method has conventionally been produced by a reaction of phosgene and methanol, but, recently, a method for producing dimethyl carbonate without phosgene has been developed and, consequently, the safety in operation is remarkably improved, and further a halide is not by-produced, and therefore this method has drawn attention as a method which causes a low load on the environment.

However, the method described in patent document 3 has a problem of the by-products of N-methylated amine and carbamate compound derived from dimethyl carbonate. For example, with respect to the method in which an aliphatic diamine and dimethyl carbonate are reacted with each other in the presence of a yttrium compound and a ytterbium compound belonging to Group 3 of the Periodic Table, it has been reported that, for preventing the formation of N-methylated compound which is the above-mentioned by-product, the reaction is conducted at a temperature of around 70°C and further a period of time of 8 hours is required until the reaction is completed, but the formation of the N-methylated compounds cannot be completely prevented.

Further, in the method described in patent document 3, methanol is by-produced by the reaction with dimethyl carbonate, and methanol and dimethyl carbonate together form an azeotrope, and therefore another problem arises in that complicated operations are needed for separation and recovery of dimethyl carbonate as a raw material and methanol after completion of the reaction. In addition, carbon dioxide generated due to decomposition of dimethyl carbonate during the reaction reacts with the catalyst to form a carbonate having no catalytic activity. Therefore, in such a reaction, the use of a large amount of the catalyst is essential for rapidly promoting the reaction. For example, in a method in which an aromatic diamine and dimethyl carbonate are reacted with each other in the presence of a tin acetate or zinc acetate

to obtain an aromatic carbamate compound, the catalyst is deactivated due to decomposition of dimethyl carbonate for the reason described above, and therefore the reaction is performed using a large amount of the catalyst and dimethyl carbonate (see, for example, patent document 5).

On the other hand, in recent years, as a method for obtaining a carbamate compound by a reaction of the N-formylated alkylenediamine compound and dimethyl carbonate, there have been reported a method characterized by a high-temperature reaction using no catalyst (see, for example, patent document 6), a method characterized by a low-temperature reaction using a small amount of an alcoholate catalyst (see, for example, patent document 7), and a method characterized by recovering the by-produced methyl formate and recycling the formamide compound as a raw material for synthesis (see, for example, patent document 8). However, the by-produced methyl formate has a boiling point of 31.5°C and a flash point of -19°C, both of which are low temperatures, and is highly toxic to human bodies, and hence special consideration is required in the operation for separation or recovery of methyl formate, for example, the operation must be performed in a closed system. Further, it is known that methyl formate decomposes into carbon monoxide and methanol in the presence of a basic compound(s), and it has been reported that when high-temperature reaction conditions are employed to improve the reaction rate, a basic compound(s) cannot be used (see, for example, patent document 9) . Therefore, the methods of patent documents 8 and 9 using dimethyl carbonate and a formamide compound are also not industrially advantageous from the viewpoint of decomposition and separation or recovery or the like of the by-product.

Patent document 1: Japanese Unexamined Patent Publication No. Hei 6-172292
Patent document 2: Japanese Unexamined Patent Publication No. Sho 55-147253
Patent document 3: Japanese Unexamined Patent Publication No. 2003-212836
Patent document 4: Japanese Unexamined Patent Publication No. 2003-252846
Patent document 5: Japanese Unexamined Patent Publication No. 2003-201275
Patent document 6: Japanese Unexamined Patent Publication No. Hei 3-200756
Patent document 7: Japanese Unexamined Patent Publication No. Hei 4-235954
Patent document 8: Japanese Unexamined Patent Publication No. Hei 10-7641
Patent document 9: Japanese Unexamined Patent Publication No. 2002-114509
Non-patent document 1: Journal of Molecular Catalysis A: Chemical 195, 37-45,2003
Non-patent document 2: Chem. Commun. 2238-2239, 2001


## DISCLOSURE OF THE INVENTION

### Problems to be Solved by the Invention

An object of the present invention is to provide a process for preparation of a carbamate compound, which is advantageous not only in that the reaction rate is faster than conventional process and the formation of methyl formate having decomposability can be prevented, but also in that the by-produced ester compound can be easily separated or recovered.

### Means to Solve the Problems

The present inventors have conducted extensive and intensive studies with a view toward solving the above-mentioned problems. As a result, it has been found that, in the process for preparation of a carbamate compound, which comprises a reaction of a specific amide compound and carbonic acid ester in the presence of a basic compound(s), the reaction proceeds more rapidly than that in the conventional method, making it possible to obtain a carbamate compound, which is a desired product, obtained in high yield in a short time, and further the formation of methyl formate having decomposability which has caused problems in the conventional method can be prevented, and furthermore both the operation for separation of the carbamate compound and the operation for recovery of the by-produced ester compound are easy, and thus the present invention has been completed.

The present invention is directed to a process for preparing a carbamate compound, which comprises reacting, in the presence of a basic compound(s), an amide compound represented by the following formula (1):

$$R^1(NHCOR^2)_n \qquad (1)$$

wherein $R^1$ represents a hydrocarbon group having 1 to 20 carbon atoms which may have a substituent(s), or a hydrocarbon group which may have a substituent(s) and may contain in the molecular skeleton thereof a linking group(s) having a heteroatom(s),

$R^2$s are the same or different and each represents a hydrocarbon group having 1 to 8 carbon atoms which may have a substituent(s), and
n represents an integer of 1 to 4,
with a carbonic acid ester represented by the following formula (2):

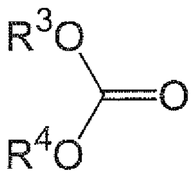

(2)

wherein $R^3$ and $R^4$ are the same or different and each represents a hydrocarbon group having 1 to 20 carbon atoms which may have a substituent(s),
to obtain a carbamate compound represented by the following formula (3):

$$R^1(NHCOOR^3)_n \qquad (3)$$

wherein $R^1$, $R^3$ and n are as defined above.

Effect of the Invention

By the process of the present invention, a carbamate compound can be easily obtained in high yield without special operations. Particularly, in the present invention, the use of a basic compound(s) improves the reaction rate which has been a problem in the conventional process, making it possible to obtain a carbamate compound in high yield in a short time. Further, in the present invention, the formation of methyl formate having decomposability which has caused problems in the conventional method can be prevented, and further both the operation for separation of the carbamate compound and it is easy to recover by-produced ester compound. Thus, the process for preparation a carbamate compound of the present invention solves the problems accompanying the conventional process and is an industrially excellent process having additional effects.

BEST MODE FOR CARRYING OUT THE INVENTION

In the present invention, in the presence of a basic compound(s), an amide compound represented by the formula (1) and a carbonic acid ester represented by the formula (2) are reacted with each other to obtain a carbamate compound represented by the formula (3)(reaction formula [1]).
Reaction formula [1]

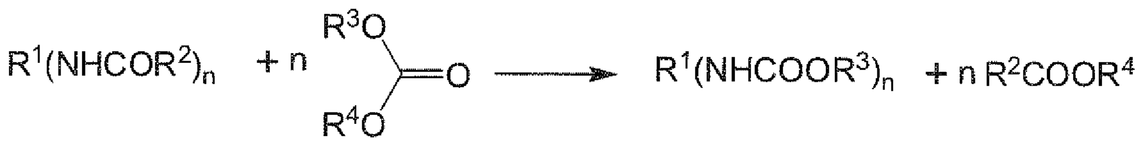

(1) (2) (3) (4)

wherein $R^1$ to $R^4$ and n are as defined above.
When a diamide compound represented by the formula (1a) is used as the amide compound of formula (1), a carbamate compound represented by the formula (3 a) is obtained in the reaction formula [1a].
Reaction formula [1a]

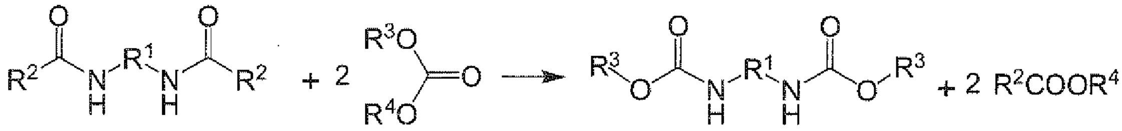

(1a) (3a)

wherein $R^1$ to $R^4$ are as defined above.

In the present invention, the amide compound as a raw material is represented by the formula (1) in the reaction formula [1]. In the formula (1), $R^1$ represents a hydrocarbon group having 1 to 20 carbon atoms which may have a substituent(s), or a hydrocarbon group which may have a substituent(s) and may contain in the molecular skeleton thereof a linking group(s) having a heteroatom(s), $R^2$s are the same or different and each represents a hydrocarbon group having 1 to 8 carbon atoms which may have a substituent(s), and n represents an integer of 1 to 4.

The hydrocarbon group in respect of $R^1$ can be a straight-chain, branched or cyclic aliphatic group, or aromatic group having 1 to 20 carbon atoms, preferably 1 to 12 carbon atoms, or a group comprising a combination of two groups or more selected from the above four classes. The valence of $R^1$ depends on n, and when n is an integer of 1 to 4, $R^1$ is a mono- to tetra-valent hydrocarbon group, respectively.

Examples of the hydrocarbon groups in respect of $R^1$ include residues of straight-chain or branched alkanes (e.g., ethane, propane, butane, pentane, hexane and dodecane), cycloalkanes (e.g., cyclopropane, cyclobutane, cyclobutane, cyclopentane and cyclohexane), 1,3-dimethylcyclohexane, 1,4-dimethylcyclohexane, polycycloalkanes (e.g., bicyclo[2.1.1]hexane, bicyclo[2.2.1]heptane, bicyclo[2.2.2]octane, bicyclo[3.3.0]octane, bicyclo[4.3.0]nonane, bicyclo [4.4.0]decane and adamantane), and aromatic hydrocarbon compounds (e.g., benzene and naphthalene).

As examples of the groups comprising a combination of two groups or more selected from the straight-chain, branched or cyclic aliphatic groups and aromatic group, there can be mentioned araliphatic groups. The araliphatic group has both an aliphatic portion(s) and an aromatic portion(s), and is a residue of, for example, benzene substituted with an alkyl group (e.g., toluene, ethylbenzene, or xylene). Examples of monovalent araliphatic groups include a benzyl group and a phenethyl group, and examples of divalent araliphatic groups include a xylylene group. These groups include their isomers.

The hydrocarbon group in respect of $R^1$ may have a substituent(s). Examples of substituents include halogen atoms, a nitro group, a cyano group, an oxo group and alkoxy groups having 1 to 6 carbon atoms (including their isomers). With respect to the above substituent, one substituent or more can be contained in the hydrocarbon group, and one type or more of the substituent may be contained.

The hydrocarbon group containing in the molecular skeleton thereof a linking group(s) having a heteroatom(s) in respect of $R^1$ is represented by the structure of $R^{1'}$ in which, for example, as shown in the formula (5) below, two hydrocarbon groups (Ra and Rb) are linked through linking group X.

$$R^{1'}=Ra\text{-}X\text{-}Rb \qquad (5)$$

wherein Ra and Rb are the same or different and are as defined above for the hydrocarbon group in respect of $R^1$, and may have a substituent(s), and
X represents a linking group(s) which may have a heteroatom(s).

The valence of $R^{1'}$ depends on n, and when n is an integer of 1 to 4, $R^{1'}$ is a mono- to tetra-valent hydrocarbon group, respectively. When $R^{1'}$ is divalent, for example, both of Ra and Rb can be divalent groups.

Linking group X is a group containing a heteroatom(s) and linking together the hydrocarbon groups (Ra and Rb) in the formula (5), and examples of linking groups include groups represented by the following formulae (6) to (24):

Linking group: -X-

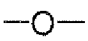 (6), 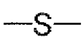 (7), 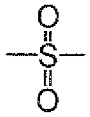 (8), 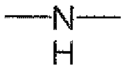 (9), 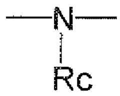 (10),

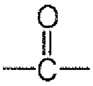 (11), 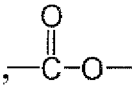 (12), 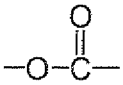 (13), 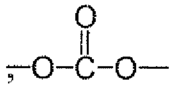 (14),

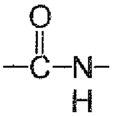 (15), 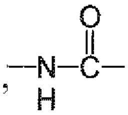 (16), 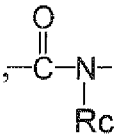 (17), 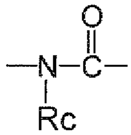 (18),

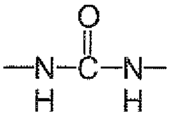 (19), 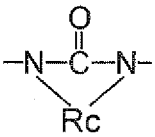 (20), 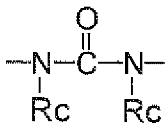 (21),

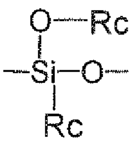 (22), 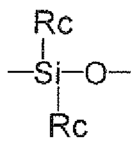 (23), 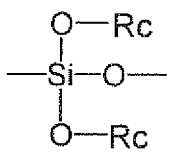 (24)

wherein Rcs are the same or different and are as defined above for the hydrocarbon group in respect of $R^1$, and may have a substituent(s).

In the formula (1), $R^2$ represents a hydrocarbon group having 1 to 8 carbon atoms which may have a substituent (s), and when n is an integer of 2 to 4, $R^2$s may be the same or different, and are preferably the same.

The hydrocarbon group in respect of $R^2$ can be a straight-chain, branched or cyclic aliphatic group, or aromatic group having 1 to 8 carbon atoms, preferably 1 to 6 carbon atoms, or a group comprising a combination of two groups or more selected from the above four classes (e.g., an araliphatic group).

The hydrocarbon group in respect of $R^2$ is preferably a straight-chain, branched or cyclic aliphatic group, further preferably a straight-chain or branched alkyl group. Also preferred are a benzyl group and a phenethyl group.

The hydrocarbon group in respect of $R^2$ may have a substituent(s). Examples of substituents include halogen atoms, a cyano group, a nitro group, an oxo group and alkoxy groups having 1 to 6 carbon atoms (including their isomers). With respect to the above substituent, one substituent or more can be contained in the hydrocarbon group, and one type or more of the substituent may be contained.

Preferred examples of $R^2$s include hydrocarbon groups unsubstituted or substituted with a halogen atom, a cyano group, or a nitro group, more preferred examples include straight-chain, branched or cyclic aliphatic groups and a benzyl group, which are unsubstituted or substituted with a fluorine atom or a chlorine atom, and especially preferred examples include straight-chain aliphatic groups having 1 to 3 carbon atoms and being unsubstituted or substituted with a fluorine atom. Especially more preferred is a methyl group and, in this case, the amide compound is represented by the following formula (25):

$$R^1(NHCOCH_3)_n \qquad (25)$$

wherein $R^1$ and n are as defined above.

In the amide compound represented by the formula (1) used in the present invention, n represents the number of the amide group(s)($NHCOR^2$), and the amide group(s)($NHCOR^2$) may be bonded to any of primary, secondary and tertiary carbon atoms. n is preferably 1 to 3, and it is more preferred that n is 1, which corresponds to the formula (26), or n is 2, which corresponds to the formula (27).

$R^1$-NHCOR$^2$ (26)

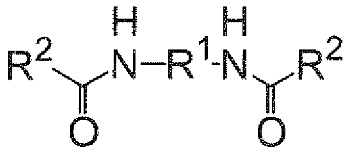

(27)

In the formulae (26) and (27), $R^1$ and $R^2$ are as defined above.

Examples of the amide compounds represented by the formula (1) used in the present invention include aliphatic amide compounds, alicyclic amide compounds, araliphatic amide compounds and aromatic amide compounds, each having 1 to 3 amide groups (NHCOR$^2$).

With respect to the amide compound represented by the formula (1) in the present invention, when n is 1 (when the compound has one amide group), examples of the amide compounds include butylacetamide, hexylacetamide and N-cyclohexylacetamide; and when n is 2 (when the compound has two amide groups), examples of the amide compounds include 1,2-ethylenebisacetamide, 1,3-propylenebisacetamide, 1,4-tetramethylenebisacetamide, 1,5-pentamethylenebisacetamide, 1,6-hexamethylenebisacetamide, 2,4,4-trimethyl-1,6-hexamethylenebisacetamide, 2,2,4-trimethyl-1,6-hexamethylenebisacetamide, 1,12-dodecamethylenebisacetamide, 1,2-cyclopropylenebisacetamide, 1,2-cyclobutylenebisacetamide, 1,3-cyclobutylenebisacetamide, 1,2-cyclopentanebisacetamide, 1,3-cyclopentanebisacetamide, 1,2-cyclohexanebisacetamide, 1,4-cyclohexanebisacetamide, 1,3-bis(acetylaminomethyl)cyclohexane and a 1,4-isomer having the same structure, 2,5-bis(acetylamino)bicyclo[2.2.1]heptane and a 2,6-structural isomer having the same structure, 2,5-bis(acetylaminomethyl)bicyclo[2.2.1]heptane and a 2,6-structural isomer having the same structure, 1,3-bis(acetylamino)cyclohexane and a 1,4-structural isomer having the same structure, 3-acetoamidomethyl-3,5,5-trimethyl-1-acetamide, bis(4-acetylaminocyclohexyl)methane and a 2-structural isomer having the same structure, 1,3-diacetylaminomethylbenzene and a 1,4-structural isomer having the same structure, bis(4-acetylaminobenzyl)methane, 1,3-bis(acetylamino)benzene and a 1,4-structural isomer having the same structure, 1,4-bis(acetylamino)-3-methylbenzene, 1,3-bis(1-acetylamino-1-methyl-ethyl)benzene and a 1,4-structural isomer having the same structure, and 1,5-naphthylenediacetamide.

In the carbonic acid ester represented by the formula (2) used in the present invention, each of $R^3$ and $R^4$ represents a hydrocarbon group having 1 to 20 carbon atoms which may have a substituent(s). $R^3$ and $R^4$ may be the same or different.

The hydrocarbon group in respect of each of $R^3$ and $R^4$ can be a straight-chain, branched or cyclic aliphatic group, or aromatic group having 1 to 20 carbon atoms, preferably 1 to 12 carbon atoms, or a group comprising a combination of two groups or more selected from the above four classes (e.g., an araliphatic group).

The hydrocarbon group in each of $R^3$ and $R^4$ is preferably a straight-chain or branched aliphatic group, or an aromatic group. As examples of straight-chain or branched aliphatic groups, there can be mentioned straight-chain or branched alkyl groups.

The hydrocarbon group in respect of each of $R^3$ and $R^4$ may have a substituent(s). Examples of substituents include halogen atoms, alkoxy groups having 1 to 6 carbon atoms (including their isomers), an amino group, dialkylamino groups having 1 to 6 carbon atoms (including their isomers), a cyano group, a nitro group and an oxo group. With respect to the above substituent, one substituent or more can be contained in the hydrocarbon group, and one type or more of the substituent may be contained.

Preferred examples of $R^3$s and $R^4$s include hydrocarbon groups unsubstituted or substituted with a fluorine atom, a chlorine atom, a cyano group, a nitro group, or an oxo group. With respect to the above substituent, one substituent or more can be contained in the hydrocarbon group, and one type or more of the substituent may be contained.

It is preferred that at least one of $R^3$ and $R^4$ is a straight-chain, branched or cyclic aliphatic group, or araliphatic group having 1 to 12 carbon atoms, and these groups may have a substituent(s).

As examples of the carbonic acid esters represented by the formula (2), there can be mentioned those of the formula (2) wherein both of $R^3$ and $R^4$ are an alkyl group having 1 to 6 carbon atoms (e.g., a methyl group, an ethyl group, or a butyl group), those of the formula (2) wherein both of $R^3$ and $R^4$ are an aryl (e.g., a phenyl group), and those of the formula (2) wherein $R^3$ and $R^4$ are a combination of an alkyl group having 1 to 6 carbon atoms (e.g., a methyl group, an ethyl group, or a butyl group) and an aryl (e.g., a phenyl group), and specific examples include carbonic acid esters, such as dimethyl carbonate, methyl ethyl carbonate, diethyl carbonate, dibutyl carbonate, diphenyl carbonate, methyl phenyl carbonate and ethyl phenyl carbonate, and especially preferred examples include dimethyl carbonate.

In the reaction formula [1] in the present invention, the carbonic acid ester represented by the formula (2) is

desirably used in an amount of the mole of the amide group in the compound represented by the formula (1){n mol when the amount of the compound represented by the formula (1) is 1 mol} or more, but the amount of the carbonic acid ester used is preferably 1.0-fold mol to 10-fold mol, more preferably 1.05-fold mol to 5-fold mol, especially preferably 1.1-fold mol to 3-fold mol based on the amide group {n mol when the amount of the compound represented by the formula (1) is 1 mol}.

In respect of the carbamate compound represented by the formula (3) obtained by the present invention, $R^1$, $R^3$ and n are as defined above.

Further, according to the present invention, in addition to the carbamate compound represented by the formula (3), an ester compound represented by the formula (4) is formed after the reaction. In respect of the ester compound represented by the formula (4), $R^2$ and $R^4$ are as defined above.

Examples of the basic compounds used in the present invention include alkali metal or alkaline earth metal compounds (e.g., hydroxides, hydrogencarbonates, carbonates and alkoxides); ion-exchanged zeolite exchanged with alkali metal or alkaline earth metal ions; oxides of an alkaline earth metal or rare earth metal;and complex oxides comprising an oxide of an alkaline earth metal and/or rare earth metal and another oxide. Further, a basic compound (s), which is formed by a reaction of the above-mentioned basic compound in the reaction system with an organic compound present in the reaction system, e.g., an alcohol or a carbonic acid ester, can also be used.

Specific examples of the alkali metal compounds used in the present invention include sodium hydroxide, potassium hydroxide, cesium hydroxide, lithium hydroxide, sodium hydrogen carbonate, sodium carbonate, potassium carbonate, cesium carbonate, lithium carbonate, and sodium, potassium, cesium, or lithium methoxide or ethoxide.

Specific examples of the alkaline earth metal compounds include magnesium hydroxide, calcium hydroxide, strontium hydroxide, barium hydroxide, magnesium hydrogen carbonate, calcium hydrogen carbonate, strontium hydrogen carbonate, barium hydrogen carbonate, magnesium or calcium methoxide or ethoxide, and magnesium methyl carbonate.

Examples of types (structures) of zeolite used in the ion-exchanged zeolite include an A type (LTA structure), an X, Y type (FAU structure), a T type (ERI, OFF structure), a mordenite type (MOR structure), and a ZMS-5 type (MFI structure). The structures of zeolite indicate zeolite structures described in Atlas of Zeolite Structure types (W. M. Meier, D. H. Olson, Ch. Baerlocher, Zeolites, 17(1/2), 1996). Zeolite has sites for exchanging cations, and examples of cations exchanged at the sites include cations of alkali metals and alkaline earth metals, such as $Li^+$, $Na^+$, $K^+$, $Ca^+$, $Cs^+$, $Mg^{2+}$, $Ca^{2+}$, $Sr^{2+}$ and $Ba^{2+}$. One type or more of these cations may be present in a mixed form.

Examples of the alkaline earth metal oxides include oxides of a metal, such as magnesium, calcium, or barium, and examples of the rare earth metal oxides include oxides of a metal, such as scandium, yttrium, a lanthanide, or an actinide.

Examples of complex oxides include silica-magnesium oxide, silica-calcium oxide, alumina-magnesium oxide and hydrotalcite.

With respect to the above basic compound, an industrially available product as such can be used, and the above basic compounds may be used individually or in combination. Further, an appropriate carrier, such as silica, alumina, titania, zirconia, or activated carbon, can be used. A carrier is used for the purpose of dispersing the catalytic active sites on its surface to achieve a catalyst having a high surface area or improving the catalyst in mechanical strength. With respect to the carrier, there is no particular limitation as long as the catalyst does not inhibit the reaction or does not hinder the catalytic activity and the catalytic activity can be exhibited in the reaction.

With respect to the basic compound used in the present invention, from the viewpoint of achieving excellent conversion of the amide compound represented by the formula (1), more preferred are magnesium oxide, a magnesium alkoxide, magnesium methyl carbonate and calcium oxide.

The amount of the basic compound used is generally 0.0001-fold mol to 10-fold mol, preferably 0.0005-fold mol to 5-fold mol, more preferably 0.001-fold mol to 2.5-fold mol, especially preferably 0.002-fold mol to less than 1-fold mol based on the amide group {n mol when the amount of the compound represented by the formula (1) is 1 mol}.

In the present invention, the carbamate compound represented by the formula (3) can also be obtained by reacting the amide compound represented by the formula (1) with the carbonic acid ester represented by the formula (2) in the presence of a Lewis acid compound(s) and a basic compound(s). When the reaction is conducted in the presence of a Lewis acid compound, the side reaction of the carbonic acid ester can be suppressed.

Examples of Lewis acid compounds include metal halides having Lewis acid properties, such as aluminum(III) bromide, aluminum(III) chloride, gallium(III) chloride, iron(III) chloride, antimony(V) chloride, tin(IV) chloride, titanium(IV) chloride, zinc(II) chloride, copper(II) chloride and magnesium(II) chloride; boron halides having Lewis acid properties, such as boron(III) fluoride and boron(III) chloride; metal carbonyl complexes, such as tris(pentafluorophenyl)borane, diphosphorus pentaoxide and $Mo(CO)_6$; and trifluoromethanesulfonates, e.g., metal triflates having Lewis acid properties, such as copper(II) trifluoromethanesulfonate and zinc(II) trifluoromethanesulfonate and lanthanoid metal triflates having Lewis acid properties, such as scandium(III) trifluoromethanesulfonate, lanthanum(III) trifluoromethanesulfonate and ytterbium(III) trifluoromethanesulfonate. These Lewis acid compounds can be used in the form of, for example, a solution

prepared using an organic solvent, or an industrially available product as such, and may be used individually or in combination.

The Lewis acid compound in the present invention is preferably a trifluoromethanesulfonate, more preferably a lanthanoid metal triflate, and is also preferably tris(pentafluorophenyl)borane.

The amount of the Lewis acid compound used is generally 0.0001-fold mol to 10-fold mol, preferably 0.0003-fold mol to 5-fold mol, more preferably 0.001-fold mol to 2.5-fold mol, especially preferably 0.002-fold mol to less than 1-fold mol based on the amide group {n mol when the amount of the compound represented by the formula (1) is 1 mol}.

The reaction in the present invention can be performed by mixing together a basic compound(s), the amide compound represented by the formula (1), and the carbonic acid ester represented by the formula (2). When a Lewis acid compound(s) is used, there is no particular limitation with respect to the timing of the addition of the compound, and the Lewis acid compound(s) may be added at the beginning of the reaction, added on the final stage of the reaction, or added successively.

The reaction in the present invention can be performed in an organic solvent(s). With respect to the organic solvent, there is no particular limitation as long as it is a solvent which does not affect the reaction. The organic solvents may be used individually or in combination. With respect to the organic solvent, a dehydrated solvent is desirably used, and examples include aliphatic hydrocarbons, such as n-pentane, n-hexane, n-heptane, n-octane, cyclopentane, cyclohexane and cycloheptane; aromatic hydrocarbons, such as benzene, toluene and xylene; ethers, such as diethyl ether, t-butyl methyl ether, diisopropyl ether, cyclopentyl methyl ether, tetrahydrofuran and 1,4-dioxane; ketones, such as acetone and methyl ethyl ketone; and acetonitrile and propionitrile.
Alternatively, by using the carbonic acid ester in an excess amount, the carbonic acid ester can be used as a solvent.

The reaction temperature in the present invention is preferably in the range between 100 to 250°C, more preferably 130 to 230°C, especially preferably 150 to 200°C. The reaction temperature is preferably is controlled so that the ester compound represented by the formula (4), which is a raw material for the by-product, can be distilled off selectively with respect to the amide compound represented by the formula (1) and the carbonic acid ester represented by the formula (2) which are raw materials and the carbamate compound represented by the formula (3) which is a product, and the reaction temperature is more preferably is controlled so that the ester compound is completely distilled off at the time when the reaction is completed.

With respect to the reaction pressure in the present invention, there is no particular limitation, and conditions under either atmospheric pressure or a pressure may be performed. The reaction can also be conducted under a reduced pressure. In this case, the reaction pressure (degree of vacuum) is preferably 0.13 kPa to less than 101.3 kPa (ImmHg to less than 760 mmHg), and further the reaction temperature is appropriately controlled so that, for example, the ester compound represented by the formula (4), which is a raw material for the by-product, can be distilled off selectively with respect to the amide compound represented by the formula (1) and the carbonic acid ester represented by the formula (2) which are raw materials and the carbamate compound represented by the formula (3) which is a product.

With respect to the manner of the reaction in the present invention, there is no particular limitation, and the reaction may be in any of a batch wise manner and a continuous flow manner, and can be conducted in any of a suspended bed, a fixed bed and a fluidized bed. The reaction time or residence time varies depending on the reaction conditions, but is generally 24 hours or less.

The obtained carbamate compound can be further purified by a general method, such as distillation, crystallization, recrystallization, separation, extraction, or column chromatography.

EXAMPLES

Hereinbelow, the present invention will be described in more detail with reference to the following Examples, which should not be construed as limiting the scope of the present invention.

The conversion rate of the amide compound and the yield of the obtained carbamate compound were determined as follows. With respect to the carbamate compound, the reaction solution was analyzed using gas chromatography having a hydrogen flame ionization detector (FID) by an internal standard method (using tridecane as an internal standard) to determine each value. With respect to the methyl dicarbamate compound, the reaction solution was analyzed using liquid chromatography having an IR detector by an absolute calibration curve method to determine each value.

Example 1 (Synthesis of methyl N-hexylcarbamate)

A glass test tube (outer diameter: 10 mm; length: 100 mm) having a cap was filled with 0.066 mmol of magnesium ethoxide, 0.025 mmol of zinc(II) trifluoromethanesulfonate, 5.00 mmol of N-hexylacetamide, 10 mmol of dimethyl carbonate, and 90 mg of tridecane as an internal standard, and a reaction was conducted at 155°C for 2 hours. After cooling to room temperature, the resultant reaction solution was analyzed by gas chromatography. As a result, it was found that the conversion of N-hexylacetamide was 85 mol%, the yield of and the selectivity for methyl N-hexylcarbamate were

80.2 mol% and 94.4 mol%, respectively, and the yield of and the selectivity for methyl acetate were 70.1 mol% and 82.5 mol%, respectively.

Comparative Example 1 (Synthesis of methyl N-hexylcarbamate)

The same procedure as in Example 1 was repeated except that the N-hexylacetamide was changed to N-hexylformamide. As a result, it was found that the conversion of N-hexylacetamide was 32.3 mol%, the yield of and the selectivity for methyl N-hexylcarbamate were 25.8 mmol% and 79.9 mol%, respectively, and the yield of and the selectivity for methyl formate were 15.9 mol% and 49.2 mol%, respectively.

The results of Example 1 and Comparative Example 1 are shown in Table 1 below.
With respect to each of the different amide compounds represented by the formula (1), a reaction rate was determined from the formula [A] below, and a reaction rate ratio between the amide compounds was determined by making a calculation from the formula [B] below.
As a result, it was found that the reaction rate ratio of Example 1 (in which N-hexylacetamide was used) to Comparative Example 1 (in which N-hexylformamide was used) was 8.2.

Formula [A]

Reaction rate (1/h) = [(Conversion rate (%))/100 (%) × (Yield (%) of desired product/100 (%)]/(Reaction time (h))

Formula [B]

Reaction rate ratio = [Reaction rate (1/h) of Example]/[Reaction rate (1/h) of Comparative Example]

[Table 1]

| | Amide compound | Upper: Basic compound<br>Lower: Lewis acid compound | | Temp. (°C) | Conversion rate (%) | | Upper: Yield (%), Lower: Selectivity (%) | | |
|---|---|---|---|---|---|---|---|---|---|
| | | | | | Amide compound | DMC | Carbamate | Ester | N-Methylated product |
| Example 1 | Hex-NHAc | $Mg(OEt)_2$<br>$Zn(OTf)_2$ | 1.3mol%<br>0.5mol% | 155 | 85.0 | 44.0 | 80.2<br>94.4 | 70.1<br>82.5 | 0.0 |
| Comparative example 1 | Hex-NHCHO | $Mg(OEt)_2$<br>$Zn(OTf)_2$ | 1.3mol%<br>0.5mol% | 155 | 32.3 | 14.0 | 25.8<br>79.9 | 15.9<br>49.2 | 0.0 |
| * N-Methylated product: N-Hexyl-N-methylacetamide | | | | | | | | | |

Example 2 (Synthesis of methyl N-hexylcarbamate)

The same procedure as in Example 1 was repeated except that the catalyst was changed to 0.06 mmol of magnesium ethoxide, and that the reaction temperature was changed to 180°C. As a result, it was found that the conversion of N-hexylacetamide was 97.7 mol%, the yield of methyl N-hexylcarbamate was 89.9 mol%, and the yield of N-hexyl-N-methylacetamide was 0.1 mol%.

Example 3 (Synthesis of N-hexyl-N-methylacetamide)

The same procedure as in Example 1 was repeated except that the catalyst was changed to 0.01 mmol of magnesium methyl carbonate, and that the reaction temperature was changed to 180°C. As a result, it was found that the conversion of N-hexylacetamide was 76.6 mol%, the yield of methyl N-hexylcarbamate was 68.6 mol%, and the yield of N-hexyl-N-methylacetamide was 0.5 mol%.

Example 4 (Synthesis of N-hexyl-N-methylacetamide)

The same procedure as in Example 1 was repeated except that the catalyst was changed to 0.06 mmol of magnesium ethoxide. As a result, it was found that the conversion of N-hexylacetamide was 71.3 mol%, the yield of methyl N-hexylcarbamate was 61.7 mol%, and the yield of N-hexyl-N-methylacetamide was 1.2 mol%.

Example 5 (Synthesis of methyl N-hexylcarbamate)

The same procedure as in Example 1 was repeated except that the magnesium ethoxide was changed to 2.8 mmol of magnesium oxide, and that the zinc(II) trifluoromethanesulfonate was changed to 0.035 mmol of ytterbium(III) trifluoromethanesulfonate. As a result, it was found that the conversion of N-hexylacetamide was 89.1 mol% and the yield of methyl N-hexylcarbamate was 84.6 mol%.

Example 6 (Synthesis of methyl N-hexylcarbamate)

The same procedure as in Example 1 was repeated except that the magnesium ethoxide was changed to 2.8 mmol of magnesium oxide, and that the zinc(II) trifluoromethanesulfonate was changed to 0.030 mmol of scandium(III) trifluoromethanesulfonate. As a result, it was found that the conversion of N-hexylacetamide was 78.8 mol% and the yield of methyl N-hexylcarbamate was 73.2 mol%.

Example 7 (Synthesis of methyl N-hexylcarbamate)

The same procedure as in Example 1 was repeated except that the zinc(II) trifluoromethanesulfonate was changed to 0.025 mmol of lanthanum(III) trifluoromethanesulfonate. As a result, it was found that the conversion of N-hexylacetamide was 97.7 mol% and the yield of methyl N-hexylcarbamate was 91.5 mol%.

Example 8 (Synthesis of methyl N-hexylcarbamate)

The same procedure as in Example 1 was repeated except that the zinc(II) trifluoromethanesulfonate was changed to 0.025 mmol) of tris(pentafluorophenyl)borane. As a result, it was found that the conversion of N-hexylacetamide was 92.0 mol% and the yield of methyl N-hexylcarbamate was 86.0 mol%.

Example 9 (Synthesis of methyl N-hexylcarbamate)

The same procedure as in Example 1 was repeated except that the N-hexylacetamide was changed to N-hexylpropionamide. As a result, it was found that the conversion of N-hexylacetamide was 68.9 mol% and the yield of methyl N-hexylcarbamate was 63.8 mol%.

Comparative Example 2 (Synthesis of methyl N-hexylcarbamate)

The same procedure as in Example 1 was repeated except that the N-hexylacetamide was changed to N-hexylformamide, and that no catalyst was added. As a result, it was found that the conversion of N-hexylacetamide was 9.6 mol% and the yield of methyl N-hexylcarbamate was 8.9 mol%.

Comparative Example 3 (Synthesis of N-hexyl-N-methylacetamide)

The same procedure as in Example 1 was repeated except that no catalyst was added. As a result, it was found that the conversion of N-hexylacetamide was 1.6 mol% and the yield of N-hexyl-N-methylacetamide was 0.9 mol%.

The results of Examples 2 to 9 and Comparative Examples 2 and 3 are shown in Table 2 below.

[Table 2]

| | Amide compound | Basic compound | | Lewis acid compound | | Temp. (°C) | Conversion rate (%) Amide compound | Yield (%) | |
|---|---|---|---|---|---|---|---|---|---|
| | | | | | | | | Carbamate | N-Methylated product |
| Example 2 | NHAc | $Mg(OEt)_2$ | 1.2mol% | - | - | 180 | 97.7 | 89.9 | 0.1 |
| Example 3 | Hex-NHAc | $Mg(OCO_2Me)(OMe)$ | 0.2mol% | - | - | 180 | 76.6 | 68.6 | 0.5 |
| Example 4 | Hex-NHAc | $Mg(OEt)_2$ | 1.2mol% | - | - | 155 | 71.3 | 61.7 | 1.2 |
| Example 5 | Hex-NHAc | MgO | 16wt% | $Yb(OTf)_3$ | 0.7mol% | 155 | 89.1 | 84.6 | 0.0 |
| Example 6 | Hex-NHAc | MgO | 16wt% | $Sc(OTf)_3$ | 0.6mol% | 155 | 78.8 | 73.2 | 0.0 |
| Example 7 | Hex-NHAC | $Mg(OEt)_2$ | 1.3mol% | $La(OTf)_3$ | 0.5mol% | 155 | 97.7 | 91.5 | 0.0 |
| Example 8 | Hex-NHAc | $Mg(OEt)_2$ | 1.3mol% | $B(C_8F_5)_3$ | 0.5mol% | 155 | 92.0 | 86.0 | 0.0 |
| Example 9 | Hex-NHCOEt | $Mg(OEt)_2$ | 1.3mol% | $Zr(OTf)_3$ | 0.5mol% | 155 | 68.9 | 63.8 | 0.0 |
| Comparative example 2 | Hex-NHCHO | - | - | - | - | 155 | 9.6 | 8.9 | 0.0 |
| Comparative example 3 | Hex-NHAc | - | - | - | - | 155 | 1.6 | 0.0 | 0.9 |
| * N-Methylated product: N-Hexyl-N-methylacetamide | | | | | | | | | |

Example 10 (Synthesis of methyl 1,6-hexamethylenedicarbamate)

A glass test tube (outer diameter: 10 mm; length: 100 mm) having a cap was filled with 0.114 g (2.83 mmol) of magnesium oxide, 0.0099 g (0.0275 mmol) of zinc(II) trifluoromethanesulfonate, 0.499 g (2.49 mmol) of hexamethylenebis(acetamide), and 0.951 g (10.6 mmol) of dimethyl carbonate, and a reaction was conducted at 155°C for 10 hours. After cooling to room temperature, the resultant reaction solution was analyzed by gas chromatography. As a result, it was found that the conversion of 1,6-hexamethylenediacetamide was 99.8%, the yield of methyl 1,6-hexamethylenedicarbamate was 83.2 mol%, and the yield of methyl 1,6-hexamethylenemonocarbamate monoacetamide was 7.8%.
Physical properties of the obtained compound (methyl 1,6-hexamethylenedicarbamate) were as follows.
HPLC Purity: 91.7% (RI)
MS Spectrum (APCI-MS); 217 $[M+H]^+$.
$^1$H-NMR Spectrum (300 MHz, $CDCl_3$) $\delta$ (ppm); 4.69 (2H, bs), 3.66 (6H, s), 3.20-3.13 (4H, m), 1.52-1.47 (8H, m), 1.36-1.31 (8H, m).

Example 11 (Synthesis of methyl 1,6-hexamethylenedicarbamate)

A glass test tube (outer diameter: 10 mm; length: 100 mm) having a cap was filled with 0.112 g (2.78 mmol) of magnesium oxide, 0.010 g (0.0275 mmol) of zinc(II) trifluoromethanesulfonate, 0.502 g (2.50 mmol) of hexamethylenebis(acetamide), and 0.905 g (10.0 mmol) of dimethyl carbonate, and a reaction was conducted at 180°C for 2 hours. After cooling to room temperature, the resultant reaction solution was analyzed by gas chromatography. As a result, it was found that the conversion of 1,6-hexamethylenediacetamide was 99.8%, the yield of methyl 1,6-hexamethylenedicarbamate was 83.9 mol%, and the yield of methyl 1,6-hexamethylenemonocarbamate mono acetamide was 8.1%.

Example 12 (Synthesis 1 of 1,3-bis(methoxycarbonylaminomethyl)cyclohexane)

In a glass flask having an internal capacity of 1,000 ml and being equipped with a stirrer, a thermometer, and a dropping funnel were placed 700 ml of chloroform, 58.4 g (738.2 mmol) of pyridine, and 50 g (351.5 mmol) of 1,3-bis (aminomethyl)cyclohexane in the form of mixture having a 7:3 diastereomer ratio, and 57.9 g (738.2 mmol) of acetyl chloride was added dropwise to the flask at 0°C over 1 hour. A reaction was conducted at 20°C for 4 hours, and the resultant suspension was concentrated under a reduced pressure to remove the solvent. To 199.0 g of the resultant white solid was added 800 ml of acetonitrile, and the solid was ground and the insoluble material was collected by filtration, and the collected material was washed with 300 ml of acetonitrile. Then, the filtrate was dried under a reduced pressure to obtain 50.7 g of 1,3-bis(acetoamidomethyl)cyclohexane having a purity of 99% or more (value obtained from an analysis by high performance liquid chromatography) in the form of white solid (isolation yield: 63.7%). Almost all the isolated product was a compound of a cis form.
A glass test tube (outer diameter: 15 mm; length: 160 mm) having a cap was filled with 0.114 g (2.83 mmol) of magnesium oxide, 0.0099 g (0.0272 mmol) of zinc(II) trifluoromethanesulfonate, 0.558 g (2.49 mmol) of the above-obtained 1,3-bis(acetoamidomethyl)cyclohexane, and 0.951 g (10.6 mmol) of dimethyl carbonate, and a reaction was conducted at 180°C for 4 hours. After cooling to room temperature, the resultant reaction solution was analyzed by high performance liquid chromatography. As a result, it was found that the conversion of 1,3-bis(acetoamidomethyl)cyclohexane was 99.4%, the yield of 1,3-bis(methoxycarbonylammomethyl)cyclohexane was 17.1 mol%, and the selectivity for 1,3-bis(methoxycarbonylaminomethyl)cyclohexane was 17.2%.
Physical properties of the obtained compound 1,3-bis(methoxycarbonylaminomethyl)cyclohexane were as follows.
HPLC Purity: 100% (Cis-Trans mixture)(UV 210 nm)
MS Spectrum (DI-MS); EI = 258, CI = 259 (M+1), 227 $[M+H]^+$.

Example 13 (Synthesis 2 of 1,3-bis(methoxycarbonylaminomethyl)cyclohexane)

A glass test tube (outer diameter: 15 mm; length: 160 mm) having a cap was filled with 0.158 g (2.83 mmol) of calcium oxide, 0.0099 g (0.0272 mmol) of zinc(II) trifluoromethanesulfonate, 0.558 g (2.49 mmol) of 1,3-bis(acetoamidomethyl)cyclohexane obtained in Example 12, and 0.951 g (10.6 mmol) of dimethyl carbonate, and a reaction was conducted at 180°C for 4 hours. After cooling to room temperature, the resultant reaction solution was analyzed by high performance liquid chromatography. As a result, it was found that the conversion of 1,3-bis(acetoamidomethyl)cyclohexane was 95.2%, the yield of 1,3-bis(methoxycarbonylaminomethyl)cyclohexane was 20.1 mol%, and the selectivity for 1,3-bis(methoxycarbonylaminomethyl)cyclohexane was 21.1%.

Example 14 (Synthesis of ethyl 1,6-hexamethylenedicarbamate)

A glass test tube (outer diameter: 15 mm; length: 160 mm) having a cap was filled with 0.114 g (2.83 mmol) of magnesium oxide, 0.0099 g (0.0272 mmol) of zinc(II) trifluoromethanesulfonate, 0.499 g (2.49 mmol) of hexamethylenebis(acetamide), and 1.247 g (10.6 mmol) of diethyl carbonate, and a reaction was conducted at 155°C for 2 hours. After cooling to room temperature, the resultant reaction solution was analyzed by high performance liquid chromatography. As a result, it was found that the conversion of 1,6-hexamethylenebisacetamide was 92.7% and the yield of ethyl 1,6-hexamethylenedicarbamate was 3.6 mol%.

Example 15 (Synthesis of phenyl 1,6-hexamethylenedicarbamate)

A glass test tube (outer diameter: 15 mm; length: 160 mm) having a cap was filled with 0.114 g (2.83 mmol) of magnesium oxide, 0.0099 g (0.0272 mmol) of zinc(II) trifluoromethanesulfonate, 0.499 g (2.49 mmol) of hexamethylenebis(acetamide), and 2.261 g (10.6 mmol) of biphenyl carbonate, and a reaction was conducted at 155°C for 2 hours. After cooling to room temperature, the resultant reaction solution was analyzed by high performance liquid chromatography. As a result, it was found that the conversion of 1,6-hexamethylenebisacetamide was 95.6% and the yield of phenyl 1,6-hexamethylenedicarbamate was 29.5 mol%.

INDUSTRIAL APPLICABILITY

The present invention is directed to a process for preparation a carbamate compound using a carbonic acid ester and an amide compound in the presence of a basic compound(s), wherein the process is a novel industrial process which is advantageous in that the reaction rate is faster than conventional process and the corresponding coproduced ester compound can be recovered.
The carbamate compound produced by the process of the present invention is a useful compound as a raw material or an intermediate in the synthesis of medical and agricultural chemicals, fine chemicals, and polymers. Particularly, the carbamate compound is a useful compound as an intermediate of an isocyanate compound which has widely been used as a raw material for polyurethane.

## Claims

**1.** A process for preparing a carbamate compound, which comprises reacting, in the presence of a basic compound (s), an amide compound represented by the following formula (1):

$$R^1(NHCOR^2)_n \qquad (1)$$

wherein $R^1$ represents a hydrocarbon group having 1 to 20 carbon atoms which may have a substituent(s), or a hydrocarbon group which may have a substituent(s) and may contain in the molecular skeleton thereof a linking group(s) having a heteroatom(s),
$R^2$s are the same or different and each represents a hydrocarbon group having 1 to 8 carbon atoms which may have a substituent(s),
and
n represents an integer of 1 to 4,
with a carbonic acid ester represented by the following formula (2):

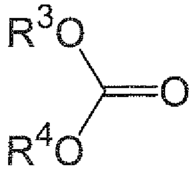

(2)

wherein $R^3$ and $R^4$ are the same or different and each represents a hydrocarbon group having 1 to 20 carbon atoms which may have a substituent(s),
to obtain a carbamate compound represented by the following formula (3):

$$R^1(NHCOOR^3)_n \quad (3)$$

wherein $R^1$, $R^3$ and n are as defined above.

2. The process according to claim 1, wherein at least one of $R^3$ and $R^4$ is a straight-chain, branched or cyclic aliphatic group, or araliphatic group having 1 to 12 carbon atoms.

3. The process according to claim 1 or 2, wherein, in the amide compound represented by the formula (1), $R^2$ is a methyl group.

4. The process according to any one of claims 1 to 3, wherein, in the amide compound represented by the formula (1), n is 1 or 2.

5. The process according to any one of claims 1 to 4, wherein the basic compound(s) is at least one member selected from the group consisting of magnesium oxide, magnesium alkoxide, magnesium methyl carbonate and calcium oxide.

6. The process according to any one of claims 1 to 5, wherein the amount of the basic compound(s) used is 0.002-fold mol to less than 1-fold mol based on the amide group in the compound represented by the formula (1).

7. The process according to any one of claims 1 to 6, which uses a Lewis acid compound(s).

8. The process according to claim 7, wherein the Lewis acid compound(s) is at least one member selected from the group consisting of trifluoromethanesulfonate and tris(pentafluorophenyl)borane.

9. The process according to claim 7 or 8, wherein the amount of the Lewis acid compound(s) used is a mole 0.002-fond mol to less than 1-fold mol based on the amide group in the compound represented by the formula (1).

10. The process according to any one of claims 1 to 9, wherein the reaction temperature is 100 to 250°C.

11. The process according to any one of claims 1 to 9, wherein the reaction pressure is 0.13 kPa to less than 101.3 kPa.

12. The process according to any one of claims 1 to 9, wherein the reaction temperature is 100 to 250°C and the reaction pressure is 0.13 kPa to less than 101.3 kPa.

13. The process according to any one of claims 1 to 12, wherein the reaction is conducted while distilling off an ester compound which is formed by the reaction and represented by the following formula (4):

$$\mathbf{R^2\text{-}COOR^4 \quad (4)}$$

wherein $R^2$ and $R^4$ are as defined in claim 1.

**Amended claims under Art. 19.1 PCT**

1. (amended) A process for preparing a carbamate compound, which comprises reacting, in the presence of an alkaline earth metal compound(s), an amide compound represented by the following formula (1):

$$\mathbf{R^1(NHCOR^2)_n \quad (1)}$$

whereon. $R^1$ represents a hydrocarbon group having 1 to 20 carbon atoms which may have a substituent(s), or a hydrocarbon group which may have a substituent(s) and may contain in the molecular skeleton thereof a linking group(s) having a heteroatom(s),
$R^2$s are the same or different and each represents a hydrocarbon group having 1 to 8 carbon atoms which may have a substituent(s),
and
n represents an integer of 1 to 4,
with a carbonic acid ester represented by the following formula (2):

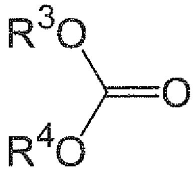

(2)

wherein $R^3$ and $R^4$ are the same or different and each represents a hydrocarbon group having 1 to 20 carbon atoms which may have a substituent(s),
to obtain a carbamate compound represented by the following formula (3):

**$R^1(NHCOOR^3)_n$ (3)**

wherein $R^1$, $R^3$ and n are as defined above.

2. The process according to claim 1, wherein at least one of $R^3$ and $R^4$ is a straight-chain, branched or cyclic aliphatic group, or araliphatic group having 1 to 12 carbon atoms.

3. The process according to claim or 2, wherein, in the amide compound represented by the formula (1), $R^2$ is a methyl group.

4. The process according to any one of claims 1 to 3, wherein, in the amide compound represented by the formula (1), n is 1 or 2.

5. The process according to any one of claims 1 to 4, wherein the alkaline earth metal compound(s) is at least one member selected from the group consisting of magnesium oxide, magnesium alkoxide, magnesium methyl carbonate and calcium oxide.

6. The process according to any one of claims 1 to 5, wherein the amount of the alkaline earth metal compound(s) used is 0.002-fold mol to less than 1 -fold mol based on the amide group in the compound represented by the formula (1).

7. The process according to any one of claims 1 to 6, which uses a Lewis acid compound(s).

8. The process according to claim 7, wherein the Lewis acid compound(s) is at least one member selected from the group consisting of trifluoromethanesulfonate and tris(pentafluorophenyl)borane.

9. The process according to claim 7 or 8, wherein the amount of the Lewis acid compound(s) used is a mole 0.002-fold mol to less than 1-fold mol based on the amide group in the compound represented by the formula (1).

10. (deleted)

11. (deleted)

12. (deleted)

13. (amended) The process according to any one of claims 1 to 9, wherein the reaction is conducted while distilling off an ester compound which is formed by the reaction and represented by the following formula (4):

**$R^2COOR^4$ (4)**

wherein $R^2$ and $R^4$ are as defined in claim 1.

## INTERNATIONAL SEARCH REPORT

International application No. PCT/JP2009/063337

A. CLASSIFICATION OF SUBJECT MATTER
*C07C269/00*(2006.01)i, *C07C271/12*(2006.01)i, *C07C271/44*(2006.01)i, *C07B61/00*(2006.01)n

According to International Patent Classification (IPC) or to both national classification and IPC

B. FIELDS SEARCHED

Minimum documentation searched (classification system followed by classification symbols)
C07C269/00, C07C271/12, C07C271/44, C07B61/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CASREACT(STN)

C. DOCUMENTS CONSIDERED TO BE RELEVANT

| Category* | Citation of document, with indication, where appropriate, of the relevant passages | Relevant to claim No. |
|---|---|---|
| X | Tetsuto T. et al., An Efficient Method for Hydrolysis of N-Monosubstituted Amides via Acetoxypivalimides, Tetrahedron Letters, 1990, 31(5), p.731-734 | 1,2,4,6, 10-13 |
| X | Tetsuto T. et al., An Efficient Method for Hydrolysis of N-Monosubstituted Amides Utilization of Intramolecular N-O Acyl Migration in Hydroxypivalimides, Tetrahedron, 1991, 47(24), p.3925-3934 | 1,2,4,6, 10-13 |
| X | Leif G. et al., Selective Cleavage of Boc-acylamides. A Novel Approach to Deacylation of Carboxamides, Acta Chem Scand Ser B, 1987, B41(1), p.18-23 | 1-4,6,10-12 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>10 August, 2009 (10.08.09) | Date of mailing of the international search report<br>18 August, 2009 (18.08.09) |
| Name and mailing address of the ISA/<br>Japanese Patent Office<br>Facsimile No. | Authorized officer<br><br>Telephone No. |

Form PCT/ISA/210 (second sheet) (April 2007)

**INTERNATIONAL SEARCH REPORT**

International application No. PCT/JP2009/063337

C (Continuation). DOCUMENTS CONSIDERED TO BE RELEVANT

| Category* | Citation of document, with indication, where appropriate, of the relevant passages | Relevant to claim No. |
|---|---|---|
| A | JP 3-200756 A (Mitsubishi Gas Chemical Co., Inc.), 02 September, 1991 (02.09.91), & US 5166414 A & EP 436800 A1 & DE 69006475 C | 1-13 |
| A | JP 4-235954 A (Mitsubishi Gas Chemical Co., Inc.), 25 August, 1992 (25.08.92), (Family: none) | 1-13 |
| A | JP 5-65263 A (Mitsubishi Gas Chemical Co., Inc.), 19 March, 1993 (19.03.93), (Family: none) | 1-13 |
| A | JP 6-228077 A (Mitsubishi Gas Chemical Co., Inc.), 16 August, 1994 (16.08.94), & EP 609786 A1 & DE 69402450 D | 1-13 |
| A | JP 10-7641 A (Mitsubishi Gas Chemical Co., Inc.), 13 January, 1998 (13.01.98), (Family: none) | 1-13 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP HEI6172292 B
- JP SHO55147253 B
- JP 2003212836 A
- JP 2003252846 A
- JP 2003201275 A
- JP HEI3200756 B
- JP HEI4235954 B
- JP HEI107641 B
- JP 2002114509 A

### Non-patent literature cited in the description

- *Journal of Molecular Catalysis A: Chemical,* 2003, vol. 195, 37-45
- *Chem. Commun.,* 2001, 2238-2239